# EUROPEAN PATENT APPLICATION

(11) **EP 2 340 819 A1**
(43) Date of publication of application: **06.07.2011**
(21) Application number: 09384008.0
(22) Date of filing: 23.12.2009
(51) Int. Cl.: A61K 31/137, A61K 31/415, A61P 25/24

(54) **Compositions comprising venlafaxine and celecoxib in the treatment of pain**

(71) Applicant: Laboratorios del. Dr. Esteve, S.A., 08041 Barcelona (ES)
(72) Inventor: Videla Cés, Sebastià, 08017 Barcelona (ES); Portillo Salido, Enrique, 08002 Barcelona (ES)
(74) Representative: Peters, Hajo

(57) **Abstract**

The present invention relates to pharmaceutical compositions comprising venlafaxine and celecoxib and their uses as medicaments, more particularly for the treatment of pain, including chronic pain; or of depression in patients which suffer from pain or in patients with a chronic musculo-skeletal inflammatory illness, with the inflammatory illness preferably being selected from osteoarthritis or rheumatoid arthritis.

## Description

The present invention relates to pharmaceutical compositions comprising venlafaxine and celecoxib and their uses as medicaments, more particularly for the treatment of pain, including chronic pain; or of depression in patients which suffer from chronic pain and/or chronic inflammation or in patients with a chronic musculo-skeletal inflammatory illness, with the inflammatory illness preferably being selected from osteoarthritis or rheumatoid arthritis.

**Pain and depression.** Psychological comorbidity is an important and frequent complication significantly changing the prognosis and course of chronic pain (Tunks et al., 2008 [1]; Kojima et al., 2009 [2]; Campbell et al., 2003 [3]). In some research articles it has been suggested that 40%-50% of the patients suffering from chronic pain suffer also from depressive disorders. Furthermore, higher levels of depression have been found to be related to a higher likelihood of experiencing pain, higher pain severity, more frequent pain and to a premature termination from pain rehabilitation programs (Banks, & Kerns, 1996 [4]; Gatchel et al., 2007 [5]). In fact, responses to chronic pain and depression appear so similar that they are often confused by medical professionals (MacDonald & Leary, 2005 [6]). This association between chronic pain and depression is not surprising since serotonin and norepinephrine, the neurotransmitters most associated with depression, play also key roles in the modulation of pain (Millan, 1999 [7]; Arnold et al., 2008 [8]).

Different chronic musculo-skeletal inflammatory Illnesses, like Rheumatoid arthritis (RA), Osteoarthritis (OA), are one of the most common reasons for seeking medical attention, sickness absence from work and long term disability. Patients suffering from this kind of illness not only suffer from the consequences of a chronic inflammation but also - sometimes increasingly over time - suffer from chronic pain symptoms.

RA is a chronic, systemic inflammatory disorder that may affect many tissues and organs, but principally attacks the joints producing an inflammatory synovitis that often progresses to destruction of the articular cartilage and ankylosis of the joints. The cause of rheumatoid arthritis is unknown; but the autoimmunity plays a pivotal role in its chronicity and progression. About 1% of the world's population is affected by rheumatoid arthritis, women three times more often than men. Onset is most frequent between the ages of 40 and 50.

OA is a flaring degenerative arthropathy and a disorder which can potentially affect all synovial joints. OA is characterised by degeneration and regeneration of articular cartilage and bone.

The pathological changes can be focal or more generalised and these changes often correlate poorly at one time point with clinical signs and symptoms but correlate longitudinally.

It is rare for OA to develop before the age of 40, but after this age the incidence increases, especially in women. OA of the hip may start a decade later than OA of the knee or hand. The prevalence of symptomatic knee OA in patients aged 35-54 years is around 1%, whereas about 40% of the population aged over 65 has symptomatic OA of the knee or hip. OA of the knee is more prevalent than hip OA.

The above mentioned inflammatory illnesses, OA and RA, can be a disabling and painful condition, which can lead to substantial loss of functioning and mobility. In fact, depression is also associated with increased functional disability in these patients. Long-term studies have shown that depression occurs following deterioration in functional ability, particularly with regard to activities which an individual regards as being important, e.g. visiting the family, going away on holiday, etc. while also the pain experienced may contribute to the overall psychological condition. For example, RA patients are twice as likely to suffer from depression as members of the general population. In RA patients, depression not only contributes its own additional burden but also interacts with the way patients perceive and cope with their physical illness and how they interact with their rheumatologist and general practitioner. Thus, depression increases the burden of RA to the patient and society.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

**Multi-Mechanism Treatment Approach**. Accordingly, evidence-based recommendations for the treatment of pain recommend paying particular attention to identify coexisting depression, anxiety, sleep disturbances, and other adverse impacts of pain on health-related quality of life and that both pain and its adverse effects should be reassessed frequently. The treatment of chronic pain and related depression is a challenging healthcare problem and targeting medications from various classes are necessary to decrease pain, improve mood, and restore normal sleep. The available drugs used as mono-treatment to treat both pain and depression syndromes have incomplete efficacy and dose-limiting adverse effects.

WO2004/060366 describes a method for the treatment of a CNS disorder, pain and inflammation in a subject in need of such treatment comprising administering to the subject a compound selected from the group consisting of duloxetine, venlafaxine and atomoxetine and a long list of COX-2 inhibitors. While trying to approach the treatment of CNS disorders like depression encountered in a patient together with inflammatory or pain symptoms the offered suggestion is a co-treatment with at least two different active agents covering a wide range of many possible combinations.

The objective of the invention was to provide new pharmacological means for the treatment of depression in connection with chronic diseases - like those with inflammatory and painful syndromes - by choosing a highly suitable combination of active compounds.

Clinical improvements/advantages desirable to the new pharmacological means would include any single one of the following or even more preferable even more than one of the following advantages below:
- Being most effective through different analgesic mechanisms providing more effective pain relief for a broader spectrum of pain.
- Providing a new more effective method for treating acute or severe to moderate pain, especially in pain with an inflammatory component.
- Reducing adverse drug reactions (side effects) while having an at least additive effect of both active compounds of the highly suitable combination.
- Reducing adverse drug reactions with a better safety profile at higher doses.
- Showing synergistic activity regarding pain and/or depression or inflammation.
- Allowing a reduction of dose while still delivering the desired activity using less of each ingredient and, therefore, reducing the side effects associated with each active principle
- Allowing optimization of the clinical management of depressive patients with an associated chronic pain condition.
- Optimizing treatment for chronic pain patients.
- Allowing treatment of patients with ongoing chronic pain and affective distress for whom traditional conservative treatments have failed.
- Enhancing the treatment adherence or compliance of patients who suffer from depression associated to chronic pain.
- Increasing the level of efficacy or reaching at lest an efficacy similar to the one achievable by each active substance used alone at higher doses associated with a better safety profile.

This objective was achieved by providing a new pharmaceutical composition comprising a combination of celecoxib and venlafaxine. Such a pharmaceutical composition seems to be very effective in the treatment of depression in connection with chronic diseases - like those with inflammatory and painful syndromes.

Therefore, the main object of the invention is a pharmaceutical composition comprising a combination of venlafaxine as a free base or as a pharmaceutically acceptable salt thereof and celecoxib.

Celecoxib is an anti-inflammatory and pain killer drug and it is one of the most used treatments for chronic musculo-skeletal inflammatory illnesses. Celecoxib, 4-[5-(4-methylphenyl)-3-(trifluoromethyl)pyrazol-1-yl]benzenesulfonamide has the following formula:

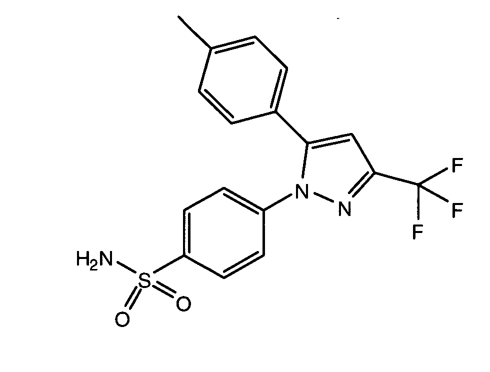

Celecoxib is an oral, highly selective cyclooxygenase-2 (COX-2) inhibitor, and it is indicated for the treatment of symptomatic relief in the treatment of osteoarthritis, rheumatoid arthritis and ankylosing spondylitis (Goldenber, 1999 [9]). This high selectivity allows celecoxib and other COX-2 inhibitors to reduce inflammation (and pain) while minimizing gastrointestinal adverse drug reactions (e.g. stomach ulcers) that are common with non-selective NSAIDs.

Cyclooxygenase is responsible for the generation of prostaglandins. Two isoforms, COX-1 and COX-2, have been identified. COX-2 is the isoform of the enzyme that has been shown to be induced by pro-inflammatory stimuli and has been postulated to be primarily responsible for the synthesis of prostanoid mediators of pain, inflammation, and fever. COX-2 is also involved in ovulation, implantation and closure of the ductus arteriosus, regulation of renal function, and central nervous system functions (fever induction, pain perception and cognitive function). It may also play a role in ulcer healing. COX-2 has been identified in tissue around gastric ulcers in man but its relevance to ulcer healing has not been established. Celecoxib inhibits COX-2 without affecting COX-1. COX-1 is involved in synthesis of prostaglandins and thromboxane, but COX-2 is only involved in the synthesis of prostaglandin. Therefore, inhibition of COX-2 inhibits only prostaglandin synthesis without affecting thromboxane and thus has no effect on platelet aggregation or blood clotting

Venlafaxine is an antidepressant drug and it is indicated for the treatment of major depressive disorder including depression accompanied by anxiety. Venlafaxine, (*rac*)-1-[2-dimethylamino-1-(4-methoxyphenyl)-ethyl]cyclohexanol, has the following formula:

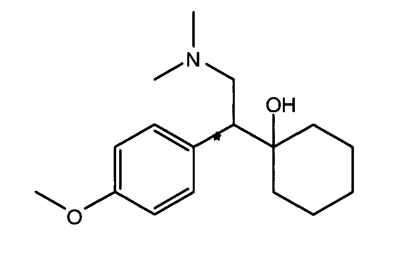

Its mechanism of action in humans is believed to be associated with its potentiation of neurotransmitter activity in the central nervous system. Preclinical studies have shown that venlafaxine and its major metabolite, O-desmethylvenlafaxine, are potent neuronal serotonin and noradrenaline re-uptake inhibitors (SNRI) and weak inhibitors of dopamine reuptake. In addition, venlafaxine and O-desmethylvenlafaxine reduce β-adrenergic responsiveness in animals after both acute (single dose) and chronic administration. Venlafaxine and its major metabolite appear to be equipotent with respect to their overall action on neurotransmitter re-uptake. Venlafaxine does contain a chiral C-atom (marked with a star) and thus may take the form of a racemate (rac)-venlafaxine or of an enantiomer ((+)-venlafaxine or (-)-venlafaxine) or mixtures thereof.

Celecoxib and venlafaxine, both have their own disadvantages when used alone or in the form of the salts known from the art. Thus, for example celecoxib is only slightly soluble in water and this is severely limiting its use in pharmaceutical formulations. On the other hand venlafaxine has many side effects. The main side effects when using venlafaxine include: anxiety; blurred vision; changes in taste; constipation; decreased sexual desire or ability; dizziness; drowsiness; dry mouth; flushing; headache; increased sweating; loss of appetite; nausea; nervousness; stomach upset; trouble sleeping; vomiting; weakness; weight loss; yawning.

As said above, the new combination shows advantages over the art.

The pharmaceutical composition according to the invention seems to show many advantages in its treatment potential like - under the proper circumstance - possibly achieving some modulation of the pharmacological effects, while also enhancing the patient's compliance.

Especially, at first impressions the pharmaceutical composition according to the invention seems to be capable of fulfilling also one or even some of the desirable clinical advantages listed above (especially if compared to any of the active principles alone), like
- providing more effective pain relief for a broader spectrum of pain,
- reducing adverse drug reactions providing a better safety profile at higher doses,
- reducing side effects with an additive effect of the compounds in the combination,
- showing synergistic activity regarding pain and or depression,
- allowing treatment of patients with ongoing chronic pain and affective distress for whom traditional conservative treatments have failed,
- enhancing the treatment adherence or compliance of patients who suffer from depression associated to chronic pain,
- increasing the level of efficacy or reaching at least an efficacy similar to the one achievable by each active substance used alone at higher doses associated with a better safety profile, or
- allowing a reduction of dose with the desired activity reducing the side effects, or
- having therapeutically activity while having a subactive dose of each active principle of the co-crystal.

In addition, it seems that in clinical practice the co-administration of venlafaxine and celecoxib in one pharmaceutical composition according to the invention would be especially useful for the often coexisting disorders depression and chronic pain, with both compounds combined showing both their analgesic and their antidepressant properties.

As said above, the main object of the invention accordingly is a pharmaceutical composition comprising a combination of venlafaxine as a free base or as a pharmaceutically acceptable salt thereof and celecoxib or a pharmaceutical composition comprising a combination of venlafaxine in neutral form or as a pharmaceutically acceptable salt thereof and celecoxib as a free base or a pharmaceutically acceptable salt thereof.

In one preferred embodiment of the pharmaceutical composition according to the invention the venlafaxine is selected from (rac)-venlafaxine, (+)-venlafaxine or (-)-venlafaxine, preferably is (rac)-venlafaxine.

In one preferred embodiment of the pharmaceutical composition according to the invention the celecoxib is in neutral form.

As celecoxib is weakly acidic with a pKa of 11.1 its "neutral form" according to the invention is defined therefore as the form in which celecoxib is free (not in form of a salt) but is - depending on the pH - neutral or carrying a load.

In one embodiment of the pharmaceutical composition according to the present invention venlafaxine (preferably (rac)-venlafaxine) and celecoxib are present in a ratio based on a fraction of their respective ED₅₀ values which ratio may vary from about 1:1 to about 1:300 or, reversibly, from about 1:1 to about 300:1.

In one further embodiment of the pharmaceutical composition according to the invention the ratio of the venlafaxine (preferably (rac)-venlafaxine) to the celecoxib is a weight ratio of from about 1:1 to about 1:300 or from about 1:1 to about 300:1.

In one further embodiment of the pharmaceutical composition according to the invention the ratio of the venlafaxine (preferably (rac)-venlafaxine) to the celecoxib is a weight ration from about 1:1 to about 1:30 or from about 1:1 to about 30:1.

In one further embodiment the pharmaceutical composition according to the invention is a pharmaceutical composition for the treatment of pain, including chronic pain; or of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis. The pain mentioned above may be chronic pain, but also severe to moderate pain.

Both parts of the pharmaceutical composition are well-known drugs used for a long time worldwide. Due to the therapeutic interest in venlafaxine in the treatment of pain symptoms and the well-known properties of celecoxib in this field of medical indication, a further object of the present invention is a medicament containing a pharmaceutical composition comprising (rac)-venlafaxine and celecoxib.

Thus, the invention also relates to a medicament containing a pharmaceutical composition comprising (rac)-venlafaxine and celecoxib according to the invention as described above and optionally one or more pharmaceutically acceptable excipients.

The medicament or pharmaceutical composition according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical compositions may for example be injected intramuscularly, intraperitoneally, or intravenously.

Medicaments or pharmaceutical compositions according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical compositions according to the present invention may also comprise an enteric coating, so that their dissolution is dependent on pH-value. Due to said coating the medicament may pass the stomach undissolved and the respective pharmaceutical composition and its components is/are liberated in the intestinal tract. Preferably the enteric coating is soluble at a pH value of 5 to 7.5. Suitable materials and methods for the preparation are known from the prior art.

Typically, the medicaments or pharmaceutical compositions according to the present invention may contain 1-60% by weight of the combination of (rac)-venlafaxine and celecoxib as defined herein and 40-99% by weight of one or more auxiliary substances (additives/excipients).

The compositions of the present invention may also be administered topically or via a suppository.

The daily dosage for humans and animals (especially adults) may vary depending on factors that have their basis in the respective species or other factors, such as age, sex, weight or degree of illness and so forth. The daily dosage for humans preferably is in the range of 5 to 800 milligrams of venlafaxine or preferably 25 to 500 to be administered during one or several intakes per day e.g, in doses of 25, 37.5, 50, 75,100, 150 or 400 mg.

The daily dosage for humans and animals (especially adults) preferably is in the range of 5 to 800 milligrams of celecoxib or preferably 50 to 500 milligrams to be administered during one or several (preferably two) intakes per day e.g, in doses of 100 or 200 mg.

The daily dosage for humans of the pharmaceutical composition according to the invention preferably is in the range in which the pharmaceutical composition is arranged to comprise between 50 to 500 milligrams of celecoxib and 25 to 500 milligrams of venlafaxine to be administered during one or several (preferably two) intakes per day e.g, in doses of 40, 50 or 100 mg of each of the two compounds or any dose in between and also doses differing between the compounds in the composition.

A further aspect of the invention relates to a pharmaceutical composition according to the invention comprising the combination of (rac)-venlafaxine.HCI and celecoxib according to the invention for use as an analgesic for the treatment of pain, including chronic pain; or of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis. Preferably this use is provided for in form of a medicament or of a pharmaceutical composition according to the invention as described above. The pain mentioned above may be chronic pain, but also severe to moderate pain.

A related further aspect of the invention is aimed at the use of a pharmaceutical composition according to the invention as described above in the manufacture of a medicament for the treatment of pain, including chronic pain; or of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis. The pain mentioned above may be chronic pain, but also severe to moderate pain.

Another object of the current invention is a method of treatment of pain, including chronic pain; or of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis, by providing to a patient in need thereof a sufficient amount of the pharmaceutical composition comprising the combination of (rac)-venlafaxine and celecoxib according to the invention as described above. The pain mentioned above may be chronic pain, but also severe to moderate pain.

The present invention is illustrated below with the help of the following examples. These illustrations are given solely by way of example and do not limit the invention.

### EXAMPLES

### Example 1

### Preparation of Composition Doses of venlafaxine and celecoxib

The preparation of different ratios of compositions comprising a combination of a venlafaxine/celecoxib are effected by preparing solutions having concentrations or suspensions expressed in mg of active drug per 10 mL of distilled water or in mg of active drug per suspension of 10 mL of 0.5% hydroxypropyl methylcellulose in distilled water. For example, 40 mg of a venlafaxine and 40 mg of celecoxib is added to a 10 mL suspension of 0.5% hydroxypropyl methylcellulose in distilled water, yielding a 10 mL suspension of a 1:1 ratio based on weight (40 mg: 40 mg). The range of doses for each ratio tested is prepared separately in a similar manner. Accordingly, other ratios of pharmaceutical compositions comprising a combination of a venlafaxine and celecoxib may also be similarly prepared at various concentrations.

### Example 2

### Formalin test

The formalin test is an analgesic behavioural observation assessment method that demonstrates two phases of nociceptive behaviour. The formalin test has an early (phase I) and a late pain related phase (phase II). Phase I seems to be caused predominantly by C-fibre activation due to the peripheral stimulus, while the phase II appears to be dependent on the combination of an inflammatory reaction in the peripheral tissue and functional changes in the dorsal horn of the spinal cord. These functional changes seem to be initiated by the C-fibre barrage during the early phase. (Dubuisson D., et al. Pain 1977, 4, 161).

### Experimental animals and housing conditions

Male CD-1 mice weighing between 20 and 25 g and male between 25 and 35g (Charles River Laboratories) are used for all the experiments. The animals are housed in a temperature-controlled room (21 ± 1 °C) with air exchange every 20 min and an automatic 12-h light/dark cycle (light from 08.00 to 20.00 h). They are fed a standard laboratory diet and tap water ad *libitum* until the beginning of the experiments. The experiments were performed during the light phase (09.00-15.00 h). Naive animals were used throughout.

### Drugs and drug administration

The mice are all injected intraperitoneally (i.p.) with compositions comprising a combination of venlafaxine and celecoxib or each agent separately, dissolved in 0.5 % hydroxypropyl methyl cellulose. The dosing volume is 10 mL/kg. Control animals received the same volume of vehicle. A concentrated formalin solution is diluted with saline to the appropriate concentration (2.5%). Formalin test is performed as described in Rosland et al., Pain 1990, 42, 235, with slight modifications.

### General procedure for assessment of pain evoked by formalin

20 microliters of the formalin solution was injected subcutaneously (s.c.) into the dorsal surface of the right hind paw of the mouse, using a Hamilton microsyringe with a 301/2-gauge needle. The time spent in licking or biting the injected paw during 45 min (divided into 9 periods of 5 min each) after the injection was measured as an indicator of the pain response.

### Example 3

### Analysis of Synergistic Effect

The analysis of possible synergistic effect for compositions comprising a combination of venlafaxine and celecoxib is determined by Plummer-Laska method (Plummer et al., Pain 1992; 49, 145 and Laska et al., Biometrics 1994, 50, 834) based on adaptations of isobolograms into simpler designs. This method allows the study of synergism using fewer data points. For the combination of agents A and B (in this case venlafaxine and celecoxib), this method compares treatment effects at three dose ratios, which can be obtained from three separate groups of animals or patients or by using a crossover design for efficiency. The first dose ratio (i) consists of agent A at a dose equal to a previous estimate of its ED50 and a zero dose of agent B. The second dose ratio (ii) consists of a zero dose of agent A and a dose of agent B equal to a previous estimate of its ED50. The third dose ratio (iii) consists of agent A in one-half the dose present in (i) and agent B in one-half the dose present in (ii). Synergism is demonstrated if the effect of (iii) is superior to both (i) and (ii). Statistical significance must be separately demonstrated in both the comparison of (iii) to (i) and the comparison of (iii) to (ii).

### Bibliography.

1. Campbell LC, Clauw DJ, Keefe FJ. (2003). Persistent pain and depression: a biopsychosocial perspective. Biol Psychiatry, 54, 399-409.
2. Kojima M, Kojima T, Suzuki S, Oguchi T, Oba M, Tsuchiya H, Sugiura F, Kanayama Y, Furukawa TA, Tokudome S, Ishiguro N. (2009). Depression, inflammation, and pain in patients with rheumatoid arthritis. Arthritis Rheum. 61, 1018-1024.
3. Tunks ER, Crook J, Weir R. (2008). Epidemiology of chronic pain with psychological comorbidity: prevalence, risk, course, and prognosis. Can J Psychiatry. 53, 224-34.
4. Gatchel RJ, Peng YB, Peters ML, Fuchs PN, Turk DC. (2007). The biopsychosocial approach to chronic pain: scientific advances and future directions. Psychol Bull. 133, 581-624
5. Banks, Sara M.; Kerns, Robert D (1996). Explaining high rates of depression in chronic pain: A diathesis-stress framework. Psychol Bull. 119, 95-110.
6. MacDonald, G., Leary, MR., (2005). Why Does Social Exclusion Hurt? The Relationship Between Social and Physical Pain. Psychol Bull. 131, 202-223.
7. Millan MJ. (1999). The induction of pain: an integrative review. Prog Neurobiol. 57, 1-164.
8. Arnold LM, Meyers AL, Sunderajan P, Montano CB, Kass E, Trivedi M, Wohlreich MM. (2008). The effect of pain on outcomes in a trial of duloxetine treatment of major depressive disorder. Ann Clin Psychiatry, 20, 187-93.
9. Goldenberg MM. (1999). Celecoxib, a selective cyclooxygenase-2 inhibitor for the treatment of rheumatoid arthritis and osteoarthritis. Clin Ther. 21, 1497-513.

## Claims

1. A pharmaceutical composition comprising a combination of venlafaxine as a free base or as a pharmaceutically acceptable salt thereof and celecoxib in neutral form or as a pharmaceutically acceptable salt thereof.

2. A pharmaceutical composition according to claim 1 wherein the celecoxib is in neutral form.

3. A pharmaceutical composition according to any of claims 1 or 2 wherein the venlafaxine is selected from (rac)-venlafaxine, (+)-venlafaxine or (-)-venlafaxine.

4. A pharmaceutical composition according to any of claims 1 to 3 wherein the ratio of the venlafaxine to the celecoxib is a weight ratio of from about 1:1 to about 1:300 and from about 1:1 to about 300:1.

5. A pharmaceutical composition according to claim 4 wherein the ratio of the venlafaxine to the celecoxib is a weight ratio of from about 1:1 to about 1:30 and from about 1:1 to about 30:1.

6. A pharmaceutical composition according to any one of claims 1 to 5 for the treatment of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis.
